(19) Europäisches Patentamt
European Patent Office
Office européen des brevets

(11) **EP 0 974 064 B1**

(12) **EUROPÄISCHE PATENTSCHRIFT**

(45) Veröffentlichungstag und Bekanntmachung des Hinweises auf die Patenterteilung:
**20.12.2000 Patentblatt 2000/51**

(21) Anmeldenummer: **97950023.8**

(22) Anmeldetag: **23.10.1997**

(51) Int Cl.[7]: **G01T 1/00**

(86) Internationale Anmeldenummer:
**PCT/EP97/05871**

(87) Internationale Veröffentlichungsnummer:
**WO 99/22252 (06.05.1999 Gazette 1999/18)**

(54) **VERFAHREN ZUR BILDERZEUGUNG BEI DER DIGITALEN DENTALEN RADIOGRAPHIE**

METHOD FOR PRODUCING IMAGES IN DIGITAL DENTAL RADIOGRAPHY

PROCEDE DE PRODUCTION D'IMAGES LORS DE LA RADIOGRAPHIE DENTAIRE NUMERIQUE

(84) Benannte Vertragsstaaten:
**AT BE CH DE FR GB LI**

(43) Veröffentlichungstag der Anmeldung:
**26.01.2000 Patentblatt 2000/04**

(73) Patentinhaber: **FRAUNHOFER-GESELLSCHAFT ZUR FÖRDERUNG DER ANGEWANDTEN FORSCHUNG E.V.
80636 München (DE)**

(72) Erfinder:
- **SCHMITT, Peter**
  **D-91058 Erlangen (DE)**
- **KOSTKA, Günther**
  **D-91056 Erlangen (DE)**
- **SCHOLZ, Oliver**
  **D-91058 Erlangen (DE)**
- **HANKE, Randolf**
  **D-90766 Fürth (DE)**
- **BAUER, Norbert**
  **D-91058 Erlangen (DE)**

(74) Vertreter: **Schoppe, Fritz, Dipl.-Ing.
Schoppe, Zimmermann & Stöckeler
Patentanwälte
Postfach 71 08 67
81458 München (DE)**

(56) Entgegenhaltungen:
**EP-A- 0 666 483        DE-A- 19 615 178
US-A- 4 914 301**

- **PATENT ABSTRACTS OF JAPAN vol. 095, no. 006, 31.Juli 1995 & JP 07 072256 A (FUJI PHOTO FILM CO LTD), 17.März 1995,**
- **PATENT ABSTRACTS OF JAPAN vol. 016, no. 357 (P-1395), 31.Juli 1992 & JP 04 110690 A (JEOL LTD), 13.April 1992,**
- **PATENT ABSTRACTS OF JAPAN vol. 095, no. 006, 31.Juli 1995 & JP 07 072254 A (FUJI PHOTO FILM CO LTD), 17.März 1995,**

**Beschreibung**

Die vorliegende Erfindung bezieht sich auf ein Verfahren zur Bilderzeugung bei der digitalen dentalen Radiographie, und insbesondere auf ein Verfahren, bei dem eine CCD-Sensoreinrichtung zur Erfassung der Röntgenstrahlung verwendet ist.

Seit längerem sind zahnärztliche Röntgendiagnostikgeräte bekannt, bei denen die Bildgewinnung unter Verwendung von CCD-Sensoren (CCD = charge coupled device = ladungsgekoppeltes Bauelement) auf digitalem Weg erfolgt. Die Bildinformation wird durch Anlegen geeigneter Taktsignale aus dem CCD-Sensor ausgelesen, vorverarbeitet, digitalisiert und schließlich zur Darstellung und Speicherung auf ein Computersystem, beispielsweise einen Personalcomputer, übertragen. Der Vorteil dieses Verfahrens gegenüber einer herkömmlichen Filmtechnik besteht vor allem in der wesentlich schnelleren Bildgewinnung.

Ein Nachteil des bekannten Verfahrens besteht jedoch darin, daß das dem Sensor aufgeprägte Bild nicht exakt wiedergegeben wird, sondern durch das sogenannte "Festmusterrauschen" (fixed pattern noise) verfälscht wird. D.h., daß bei gleicher Strahlungsintensität der digitale Grauwert eines Bildelements, d.h. eines Pixels, aufgrund fertigungsbedingter Unterschiede zum Teil erheblich von Pixel zu Pixel schwankt. Wie die Bezeichnung "Festmusterrauschen" andeutet, wirken sich die fertigungsbedingten Unterschiede für den Betrachter des Bildes zum Teil wie ein zusätzlicher Rauschanteil aus, obwohl diese nicht stochastischer Natur sind. Die Ursachen des Festmusterrauschens sind zum Teil darin zu finden, daß die einzelnen Pixel oder Elemente eines CCD-Sensor-Arrays fertigungsbedingt eine Streuung sowohl des Dunkelsignals als auch der Effizienz der Lichtwandlung, d.h. der Umsetzung des einfallenden Lichtes in ein elektrisches Signal, aufweisen.

In den meisten Fällen wird die einfallende Röntgenstrahlung nicht direkt durch den CCD-Sensor in ein elektrisches Signal umgewandelt. Vielmehr befindet sich auf der Oberseite des Sensors eine Szintillatorschicht, die eine einfallende Röntgenstrahlung in ein sichtbares Licht umwandelt, welches wiederum von dem CCD-Sensor in ein elektrisches Signal umgewandelt wird.

Inhomogenitäten in der Szintillatorschicht führen dazu, daß das Bild bei homogener Bestrahlung keinen gleichmäßigen Grauwert aufweist, sondern "fleckig" wirkt. Bei Röntgenaufnahmen von Zähnen können diese "Flecken" die Diagnose negativ beeinflussen, da sie vom Zahnarzt fälschlicherweise z.B. als Karies gedeutet werden können.

Um den Einfluß dieser fertigungsbedingten Unterschiede des Dunkelsignals und des Wirkungsgrades der Lichtumwandlung zwischen den Pixeln eines Sensor-Arrays, sowie von Inhomogenitäten der Szintillatorschicht zu minimieren, werden bei bekannten zahnärztlichen Röntgendiagnostikgeräten relativ hohe Dosen einer Röntgenstrahlung verwendet. Dadurch wird auch der Einfluß einer Temperaturabhängigkeit des Dunkelsignals und des Wirkungsgrades der Lichtumwandlung der einzelnen Pixel auf das erzeugte Bild reduziert. Selbstverständlich ist es erwünscht, bei der zahnärztlichen Röntgendiagnose geringere Strahlungsdosen zu verwenden, um die gesundheitliche Belastung der Patienten zu reduzieren.

In den Patent Abstracts of Japan, Vol. 95, Nr. 006, 31. Juli 1995, und der zugehörigen JP 07 072256 A ist eine Schaltung zur Herausfilterung des Bildrauschens aufgrund kapazitiver Fehler der Ausleseverstärker eines Halbleitersensorarrays für die Erfassung von Röntgenstrahlen beschrieben. Dabei werden zunächst jeweils Signale erfaßt, wenn das jeweilige Sensorelement nicht mit einer Röntgenstrahlung bzw. mit einer bekannten Röntgenstrahlung beaufschlagt ist, wobei diese Signale in einer Korrekturtabelle gespeichert werden. Nachfolgend wird eine Offset- und Gain-Korrektur von erfaßten Bildsignalen auf der Grundlage der Korrekturtabelle durchgeführt.

Die Patent Abstracts of Japan, Vol. 16, Nr. 357 (P-1395), 31. Juli 1992, und die dazugehörige JP 04 110690 A offenbaren eine Temperatursteuerung für ein Halbleitersensorarray zur Erfassung von Röntgenstrahlung, gemäß der die Temperatur des Erfassungsbereichs des Halbleitersensorarrays auf eine konstante Temperatur gesteuert wird.

Ausgehend von dem genannten Stand der Technik besteht die Aufgabe der vorliegenden Erfindung darin, ein Verfahren zur Bilderzeugung bei der digitalen dentalen Radiographie zu schaffen, bei dem Qualitätsminderungen des erzeugten Bildes, die durch fertigungsbedingte Unterschiede des Dunkelsignals und des Wirkungsgrades der Lichtumwandlung der einzelnen Sensorelemente sowie eine Inhomogenität der Szintillatorschicht bewirkt werden, beseitigt sind.

Diese Aufgabe wird durch ein Verfahren gemäß Patentanspruch 1 gelöst.

Das erfindungsgemäße Verfahren basiert auf der Anwendung einer pixelweisen Korrektur der elektrischen Signale, die durch jedes Element einer Sensoreinrichtung erzeugt werden, um die Bildqualität zu optimieren, indem unter anderem Schwankungen erfaßter Bildsignale aufgrund von Inhomogenitäten der Szintillatorschicht kompensiert werden.

Um diese Kompensation zu erreichen, werden Referenzsignale erfaßt, die den Ausgangssignalen des CCD-Arrays entsprechen, wenn die Sensoreinrichtung keiner Röntgenstrahlung bzw. einer homogenen Röntgenstrahlung ausgesetzt ist. Auf der Basis dieser Referenzsignale werden die elektrischen Signale, die einem Bild eines Objekts entsprechen, korrigiert. Während des Erfassens der Referenzsignale und der dem Bild eines Objekts entsprechenden Signale wird die Sensoreinrichtung auf einer konstanten Temperatur gehalten. Dadurch kann der Einfluß der Temperaturabhängigkeit des Dunkelstroms bzw. des Umwandlungswirkungsgrades der einzelnen Pixelemente auf das resultierende Bild beseitigt werden.

Die vorliegende Erfindung schafft somit ein Verfahren zur Bilderzeugung bei der digitalen dentalen Radiographie, mit dem trotz einer verringerten Röntgendosis qualitativ hochwertige Bilder erzeugt werden können, bei denen eine Verschlechterung der Bildqualität aufgrund fertigungsbedingter Unterschiede des Dunkelstroms und des Umwandlungswirkungsgrads der einzelnen Bildelemente der Sensoreinrichtung als auch aufgrund von Inhomogenitäten der Szintillatorschicht verhindert ist.

Im folgenden wird ein Ausführungsbeispiel der vorliegenden Erfindung näher erläutert.

Wie oben beschrieben wurde, beeinflussen fertigungsbedingte Unterschiede sowohl das Dunkelsignal eines Bildelements, d.h. den Grauwert des Pixels ohne eine Bestrahlung, als auch den Umwandlungswirkungsgrad bei einer vorgegebenen Bestrahlung, d.h. den Grauwert des Pixels bei der vorgegebenen Bestrahlung. Das von CCD-Sensoren erzeugte elektrische Signal hängt weitgehend linear von der Beleuchtungsstärke ab. Der digitale Grauwert $Gw_{ij}$ eines Pixels in einer Zeile i und einer Spalte j eines Sensor-Arrays kann bei vorgegebener Bestrahlungsstärke daher durch die folgende Gleichung beschrieben werden:

$$Gw_{ij}(I)=O_{ij}+G_{ij}I \qquad (1)$$

wobei $Gw_{ij}$ der Grauwert ist, $O_{ij}$ der Versatz (der Offset) ist, der durch das Dunkelsignal bewirkt wird, $G_{ij}$ der Gewinn (Gain) ist, und I die Intensität der Bestrahlung ist. $O_{ij}$ und $G_{ij}$ können sich von Pixel zu Pixel unterscheiden.

Ein weiteres Problem folgt aus der Tatsache, daß der Versatz $O_{ij}$ stark temperaturabhängig ist, wodurch der Grauwert eines Bildelements nicht nur von der Strahlungsstärke, sondern ferner von der Temperatur abhängt:

$$Gw_{ij}(T,I)=O_{ij}(T)+G_{ij}I \qquad (2)$$

Zur Korrektur des Festmusterrauschens (fixed pattern noise) für eine fest vorgegebene Temperatur T werden für jedes Pixel, d.h. für jedes Sensorelement des Sensor-Arrays, die Werte für $O_{ij}$ und $G_{ij}$ bestimmt. Zur Bestimmung derselben werden zwei Bilder benötigt: ein Bild ohne Bestrahlung sowie ein Bild bei einer bekannten Bestrahlung. Die bekannte Bestrahlung kann beispielsweise die maximal zulässige Bestrahlung sein.

Diese Bilder bei fehlender Bestrahlung und einer bekannten Bestrahlung werden zur Unterdrückung des quantenbedingten Bildrauschens vorteilhafterweise durch Mittelung mehrerer Aufnahmen unter konstanten Bedingungen erzeugt. Aus den vorzugsweise gemittelten Bildern sind somit für jedes Bildelement der Grauwert ohne bzw. mit bekannter Bestrahlung I\* bekannt:

$$Gw_{ij}(T,I=O)=O_{ij}(T) \qquad (3)$$

$$Gw_{ij}(T,I=I^*)=O_{ij}(T)+G_{ij}I^* \qquad (4)$$

$Gw_{ij}(T,I=O)$ ist der Grauwert des Pixels in der Zeile i und der Spalte j des Sensors, wenn die Sensoreinrichtung keiner Röntgenstrahlung durch eine Röntgenstrahlungsquelle ausgesetzt ist. Dieser Grauwert entspricht dem temperaturabhängigen Versatz $O_{ij}(T)$ des Pixels. $Gw_{ij}(T,I=I^*)$ ist der Grauwert des Pixels bei einer Röntgenstrahlung mit der Intensität I\*. Dieser Grauwert setzt sich aus dem Versatz $O_{ij}$ und der Intensität I\* mal dem Gewinn $G_{ij}$ des Pixels zusammen.

Durch eine pixelweise Subtraktion der Gleichung 4 minus die Gleichung 3 erhält man den Grauwert des Pixels ohne Versatzanteile:

$$Gw'_{ij}(T,I=I^*)=Gw_{ij}(T,I=I^*)-Gw_{ij}(T,I=O)=G_{ij}I^* \qquad (5)$$

Um mittels eines Sensor-Arrays ein qualitativ hochwertiges Bild zu erzeugen, müssen alle Pixel, d.h. Sensorelemente, des Arrays denselben Grauwert $Gw_{max}$ aufweisen, wenn sie mit derselben maximal zulässigen Dosis bestrahlt werden. Unterschiede in der Empfindlichkeit können durch eine Normierung der Werte $G_{ij}$ beseitigt werden. Aus der Gleichung

$$Gw_{max}=Gw'_{ij}(T,I=I^*)G_{ij}' \qquad (6)$$

kann der normierte Gewinn $G_{ij}'$ für jedes Pixel berechnet werden. $Gw_{max}$ stellt dabei einen Soll-Wert bei einer maximalen Bestrahlung dar, den sämtliche Pixel des Sensor-Arrays aufweisen sollen. Der Korrekturwert $G_{ij}'$ gibt somit einen Faktor an, mit dem der Grauwert jedes Pixels multipliziert werden muß, damit für jedes Pixel bei einer einheitlichen Bestrahlung aller Sensorelemente der gleiche Grauwert ausgegeben wird.

Die Korrektur eines durch die Grauwerte $Gw_{ij}$ charakterisierten Bildes erfolgt wie folgt: die korrigierten Grauwerte $Gw'_{ij}$ ergeben sich durch pixelweise Subtraktion der Versatzwerte $O_{ij}$ jedes Pixels von den ein Bild darstellenden Grauwerten jedes Pixels und die anschließende pixelweise Multiplikation mit den Gewinn-Normierungswerten $G_{ij}'$.

$$Gw'_{ij}=(Gw_{ij}-O_{ij})G_{ij}' \qquad (7)$$

Die Gleichungen (1) bis (7) stellen die Grundlage für die Verbesserung der Bildqualität bei der Vorrichtung und dem Verfahren gemäß der vorliegenden Erfindung dar. Diese Gleichungen werden gemäß der vorliegenden Erfindung zur pixelweisen Gewinn- und Versatz-Korrektur im Bereich der zahnärztlichen digitalen Radiographie verwendet.

Wie oben bereits dargelegt wurde, sind die Versatzwerte $O_{ij}$ und damit auch die Gewinn-Normierungswerte $G_{ij}'$ stark temperaturabhängig. Werden die Versatzwerte und die Gewinn-Normierungswerte bei einer bestimmten Temperatur ermittelt, das zu korrigierende Bild jedoch bei einer anderen Temperatur aufgenommen, so wird die Bildqualität nicht verbessert, sondern vielmehr mit großer Wahrscheinlichkeit verschlechtert. Aus diesem Grund muß die Temperatur des CCD-Sensors während der Erfassung der Referenzsignale und der Erfassung der ein Bild darstellenden Signale konstant gehalten werden. Eine konstante Sensortemperatur ist auf unterschiedliche Arten realisierbar.

Der CCD-Sensor kann auf der Rückseite mit einem Heizelement versehen werden, wobei die Temperatur des Sensors durch eine aktive Regelung konstant gehalten wird. Zur Messung der Temperatur können beispielsweise Temperatursensoren zusätzlich auf den Träger des CCD-Sensors aufgebracht sein oder auch in dem CCD-Sensor integriert sein. Falls auf dem Sensor unbelichtete CCD-Elemente, sogenannte "dark reference pixel", aufgebracht sind, können diese Elemente aufgrund der starken Temperaturabhängigkeit des Dunkelstroms zur Temperaturmessung herangezogen werden.

Ferner kann die Temperatur gegebenenfalls durch eine Steuerung der Taktfrequenz des CCD-Sensors gesteuert werden. Auch in diesem Fall kann die Temperatur der Sensoreinrichtung beispielsweise mittels Temperatursensoren, die zusätzlich auf den Träger des CCD-Sensors aufgebracht sind, erfaßt werden, um eine geregelte Steuerung der Temperatur der Sensoreinrichtung zu ermöglichen.

Bei intraoralen Röntgendiagnostikeinrichtungen kann das Sensorelement zwischen zwei Röntgenaufnahmen beispielsweise in einem Wasserbad auf einer vorbestimmten Temperatur gehalten werden. Diese vorbestimmte Temperatur kann beispielsweise die Körpertemperatur sein. Dadurch ist gewährleistet, daß das Sensorelement zwischen den Röntgenaufnahmen nicht abkühlt.

Eine weitere wesentliche Voraussetzung für den erfolgreichen Einsatz der Verstärkungs-/Versatz-Korrektur besteht darin, daß die relative Position der Sensoreinrichtung gegenüber der Röntgenstrahlungsquelle konstant ist, oder daß zumindest gewährleistet ist, daß die Röntgendosisleistung über die gesamte Sensorfläche konstant ist, um einen Abfall der Bildhelligkeit zum Bildrand hin, ein sogenanntes "Shading", zu vermeiden. Weist die Sensoreinrichtung bezüglich der Röntgenstrahlungsquelle keine konstante Bestrahlungsgeometrie auf, wird die Gewinnkorrektur nicht korrekt durchgeführt, wodurch ein künstliches Schattieren (shading) erzeugt wird. Zur Vermeidung dieser Problematik kann beispielsweise eine einem Filmhalter analoge Anordnung, bei der die Mitte des CCD-Sensors vom Zentralstrahl der Röntgenstrahlungsquelle getroffen wird, verwendet werden.

Die vorliegende Erfindung schafft somit eine Optimierung der Bildqualität im Bereich der digitalen dentalen Radiographie durch die Verwendung einer pixelweisen Gewinn- und Versatz-Korrektur bei einer konstanten Temperatur der Sensoreinrichtung. Die pixelweise Gewinn-/Versatz-Korrektur beseitigt sowohl die Folgen fertigungsbedingter Unterschiede in Dunkelstrom und Wandlungseffizienz der einzelnen Bildelemente, als auch die Folgen von Inhomogenitäten in der auf dem CCD-Sensor aufgebrachten Szintillatorschicht. Eine Voraussetzung für die Verbesserung der Bildqualität durch eine pixelweise Gewinn-/Versatz-Korrektur sind (a) eine konstante Temperatur des CCD-Sensors und (b) eine konstante Bestrahlungsgeometrie oder eine homogene Bestrahlung. Die vorliegende Erfindung ermöglicht somit die Erzeugung qualitativ hochwertiger Bilder mit gegenüber üblicherweise verwendeten Röntgenstrahlungsdosen geringen Bestrahlungsintensitäten.

Anstelle des beschriebenen CCD-Arrays kann auch ein Photodiodenarray oder ein charge-injection-device oder ein CMOS-Bildsensorarry verwendet werden.

## Patentansprüche

**1.** Verfahren zur Bilderzeugung bei der digitalen dentalen Radiographie mit folgenden Schritten:

a) Erfassen erster Referenzsignale, die durch die Elemente einer Sensoreinrichtung, die aus einem mit einer Szintillatorschicht versehenen CCD-Array besteht, erzeugt werden, wenn die Sensoreinrichtung keiner durch eine Röntgenstrahlungsquelle erzeugten Röntgenstrahlung ausgesetzt ist;

b) Erfassen zweiter Referenzsignale, die durch die Elemente der Sensoreinrichtung erzeugt werden, wenn die Sensoreinrichtung einer bekannten Röntgenstrahlung von der Röntgenstrahlungsquelle ausgesetzt ist;

c) Erfassen dritter elektrischer Signale, die durch die Elemente der Sensoreinrichtung erzeugt werden, wenn die Sensoreinrichtung einer ein Bild eines Objekts darstellenden Röntgenstrahlung ausgesetzt ist;

d) Steuern der Temperatur der Sensoreinrichtung während der Schritte a) bis c) auf eine konstante Temperatur;

e) Korrigieren der dritten elektrischen Signale, die im Schritt c) erfaßt werden, auf der Basis der ersten und zweiten Referenzsignale, die in den Schritten a) und b) erzeugt werden, um Schwankungen der dritten elektrischen Signale der einzelnen Elemente der Sensoreinrichtung aufgrund des Dunkelstroms derselben, aufgrund eines unterschiedlichen Umwandlungswirkungsgrads derselben und aufgrund von Inhomogenitäten der Szintillatorschicht zu kompensieren.

2. Verfahren gemäß Anspruch 1, bei dem die ersten Referenzsignale im Schritt a) erfaßt werden, indem mehrere Aufnahmen der Sensoreinrichtung, während dieselbe keiner durch die Röntgenstrahlungsquelle erzeugten Röntgenstrahlung ausgesetzt ist, gemittelt werden.

3. Verfahren gemäß Anspruch 1 oder 2, bei dem die zweiten Referenzsignale im Schritt b) erfaßt werden, indem mehrere Aufnahmen der Sensoreinrichtung, während dieselbe einer bekannten Röntgenstrahlung von der Röntgenstrahlungsquelle ausgesetzt ist, gemittelt werden.

4. Verfahren gemäß einem der Ansprüche 1 bis 3, bei dem das erste Referenzsignal jedes Elements der Sensorvorrichtung jeweils als Versatzwert ($O_{ij}$) für dieses Element verwendet wird, und bei dem auf der Basis eines SollWerts der elektrischen Signale der Elemente der Sensoreinrichtung bei der bekannten Röntgenstrahlung und den ersten und den zweiten Referenzsignalen ein Gewinn-Normierungswert ($G_{ij}'$) für jedes Element der Sensoreinrichtung berechnet wird.

5. Verfahren gemäß Anspruch 4, bei dem die dritten elektrischen Signale, die das Bild eines Objekts darstellen, korrigiert werden, indem der Versatzwert ($O_{ij}$) für jedes Element von dem dritten elektrischen Signal für dieses Element subtrahiert wird, und indem das Ergebnis der Subtraktion mit dem Gewinn-Normierungswert ($G_{ij}'$) für jedes Element multipliziert wird.

6. Verfahren gemäß Anspruch 4 oder 5, bei dem der Gewinn-Normierungswert ($G_{ij}'$) für jedes Element berechnet wird, indem jeweils das erste Referenzsignal von dem zweiten Referenzsignal subtrahiert wird, und indem der Soll-Wert bei der bekannten Röntgenstrahlung durch das Subtraktionsergebnis geteilt wird.

7. Verfahren gemäß einem der Ansprüche 1 bis 6, bei dem die bekannte Röntgenstrahlung die maximal zulässige Röntgenstrahlung für die Sensoreinrichtung ist.

8. Verfahren gemäß einem der Ansprüche 1 bis 7, bei dem die Schritte c) und e) wiederholt durchgeführt werden, wobei die Sensoreinrichtung zwischen aufeinanderfolgenden Schritten c) in ein Fluid einer vorbestimmten Temperatur eingetaucht wird.

**Claims**

1. A method for producing images in digital dental radiography, comprising the steps of:

a) detecting first reference signals generated by the elements of a detector device, consisting of a CCD array provided with a scintillator layer, when the detector device is not exposed to any X-radiation generated by a source of X-radiation;

b) detecting second reference signals generated by the elements of the detector device when the detector device is exposed to a known X-radiation from the source of X-radiation;

c) detecting third electrical signals generated by the elements of the detector device when the detector device is exposed to X-radiation representing an image of an object;

d) controlling the temperature of the detector device during steps a) to c) so as to maintain a constant temperature;

e) correcting the third electrical signals, detected in step c), on the basis of the first and second reference signals generated in steps a) and b) so as to compensate fluctuations in the third electrical signals of the individual elements of the CCD array caused by the dark current of these array elements, by the variation in the conversion efficiency of these array elements and by the inhomogeneity of the scintillator layer.

2. A method according to claim 1, wherein the first reference signals are detected in step a) by averaging several pictures of the detector device while this is not exposed to any X-radiation generated by the source of X-radiation.

3. A method according to claim 1 or 2, wherein the second reference signals are detected in step b) by averaging several pictures of the detector device while this is exposed to a known X-radiation from the source of X-radiation.

**4.** A method according to one of the claims 1 to 3, wherein the first reference signal of each element of the detector device is used as the offset value $(O_{ij})$ for the element concerned and wherein a gain normalization value $(G_{ij}')$ is calculated for each element of the detector device on the basis of a nominal value of the electrical signals of the elements of the detector device at the known X-radiation and the first and the second reference signals.

**5.** A method according to claim 4, wherein the third electrical signals representing the image of an object are corrected by subtracting the offset value $(O_{ij})$ for each element from the third electrical signal for the element concerned and by multipying the result of the subtraction by the gain normalization value $(G_{ij}')$ for each element.

**6.** A method according to claim 4 or 5, wherein the gain normalization value $(G_{ij}')$ for each element is calculated by subtracting the respective first reference signal from the respective second reference signal and by dividing the nominal value at the known X-radiation by the result of the subtraction.

**7.** A method according to one of the claims 1 to 6, wherein the known X-radiation is the maximum permissible X-radiation for the detector device.

**8.** A method according to one of the claims 1 to 7, wherein steps c) and e) are performed repeatedly and where the detector device is immersed in a fluid at a predetermined temperature between successive steps c).

**Revendications**

**1.** Procédé de production d'images lors de la radiographie dentaire numérique, aux étapes suivantes consistant à :

a) saisir des premiers signaux de référence générés par les éléments d'un dispositif de détection consistant en une rangée de CCD munis d'une couche scintillante, lorsque le dispositif de détection n'est pas exposé à des rayons X produits par une source de rayons X ;
b) saisir des seconds signaux de référence générés par les éléments du dispositif de détection, lorsque le dispositif de détection est exposé à des rayons X connus de la source de rayons X;
c) saisir des troisièmes signaux électriques générés par les éléments du dispositif de détection, lorsque le dispositif de détection est exposé à des rayons X représentant une image d'un objet ;

d) réguler la température du dispositif de détection au cours des étapes a) à c) à une température constante ;
e) corriger les troisièmes signaux électriques, saisis à l'étape c), sur base des premiers et seconds signaux de référence générés aux étapes a) et b), afin de compenser les variations des troisièmes signaux électriques des différents éléments du dispositif de détection sur base du courant d'obscurité de ces derniers, sur base d'un degré d'efficacité de transformation différent de ces derniers et sur base de non-homogénéités de la couche scintillante.

**2.** Procédé suivant la revendication 1, dans lequel les premiers signaux de référence sont saisis à l'étape a) en établissant la moyenne de plusieurs prises de vue du dispositif de détection tandis que celui-ci n'est pas exposé à des rayons X produits par la source de rayons X.

**3.** Procédé suivant la revendication 1 ou 2, dans lequel les seconds signaux de référence sont saisis à l'étape b) en établissant la moyenne de plusieurs prises de vue du dispositif de détection tandis que celui-ci est exposé à des rayons X connus de la source de rayons X.

**4.** Procédé suivant l'une des revendications 1 à 3, dans lequel le premier signal de référence de chaque élément du dispositif de détection est chaque fois utilisé comme valeur de décalage $(O_{ij})$ de cet élément, et dans lequel est calculée, sur base d'une valeur de consigne des signaux électriques des éléments du dispositif de détection pour les rayons X connus et les premiers et seconds signaux de référence, une valeur de normalisation de gain $(G_{ij}')$ de chaque élément du dispositif de détection.

**5.** Procédé suivant la revendication 4, dans lequel les troisièmes signaux électriques représentent l'image d'un objet sont corrigés en soustrayant la valeur de décalage $(O_{ij})$ de chaque élément du troisième signal électrique de cet élément et en multipliant le résultat de la soustraction par la valeur de normalisation de gain $(G_{ij}')$ de chaque élément.

**6.** Procédé suivant la revendication 4 ou 5, dans lequel la valeur de normalisation de gain $(G_{ij}')$ de chaque élément est calculée en soustrayant chaque fois le premier signal de référence du second signal de référence et en divisant la valeur de consigne pour les rayons X connus par le résultat de la soustraction.

**7.** Procédé suivant l'une des revendications 1 à 6, dans lequel les rayons X connus sont les rayons X maximaux admissibles pour le dispositif de détection.

8. Procédé suivant l'une des revendications 1 à 7, dans lequel les étapes c) et e) sont réalisées de manière répétée, le dispositif de détection étant, entre étapes c) successives, plongé dans un fluide à une température prédéterminée.